(19) Europäisches Patentamt
European Patent Office
Office européen des brevets

(11) **EP 2 846 201 B1**

(12) **FASCICULE DE BREVET EUROPEEN**

(45) Date de publication et mention
de la délivrance du brevet:
**27.04.2016 Bulletin 2016/17**

(21) Numéro de dépôt: **14183064.6**

(22) Date de dépôt: **01.09.2014**

(51) Int Cl.:
**G03H 1/00** *(2006.01)*     **G01N 21/35** *(2014.01)*
**G03H 1/08** *(2006.01)*     **G03H 1/22** *(2006.01)*
**H01L 31/055** *(2014.01)*

(54) **Dispositif émissif lumineux à structures diffractives et à hologramme synthétique**

Leuchtvorrichtung mit Beugungsstrukturen und synthetischem Hologramm

Light-emitting device with diffractive structures and synthetic hologram

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **05.09.2013 FR 1358492**

(43) Date de publication de la demande:
**11.03.2015 Bulletin 2015/11**

(73) Titulaire: **Commissariat à l'Énergie Atomique
et aux Énergies Alternatives
75015 Paris (FR)**

(72) Inventeur: **Martinez, Christophe
38100 Grenoble (FR)**

(74) Mandataire: **Brevalex
95, rue d'Amsterdam
75378 Paris Cedex 8 (FR)**

(56) Documents cités:
• **IRINA PUSCASU: "Photonic crystals enable
infrared gas sensors", PROCEEDINGS OF SPIE,
vol. 5515, 1 janvier 2004 (2004-01-01), pages
58-66, XP055011700, ISSN: 0277-786X, DOI:
10.1117/12.559644**
• **HASMAN E ET AL: "Manipulation of thermal
emission by use of micro and nanoscale
structures", JOURNAL OF HEAT TRANSFER,
NEW YORK, NY, US, vol. 134, no. 3, 1 mars 2012
(2012-03-01), pages 31023/1-7, XP009178412,
ISSN: 0022-1481**
• **MARQUIER F ET AL: "Coherent spontaneous
emission of light by thermal sources", PHYSICAL
REVIEW. B, CONDENSED MATTER AND
MATERIALS PHYSICS, AMERICAN INSTITUTE
OF PHYSICS, WOODBURY, NY, US, vol. 69, no.
15, 15 avril 2004 (2004-04-15), pages 155412-1,
XP002353244, ISSN: 1098-0121, DOI:
10.1103/PHYSREVB.69.155412**
• **PIERRE BARRITAULT ET AL: "Mid-IR source
based on a free-standing microhotplate for
autonomous COsensing in indoor applications",
SENSORS AND ACTUATORS A, ELSEVIER
SEQUOIA S.A., LAUSANNE, CH, vol. 172, no. 2,
21 septembre 2011 (2011-09-21), pages 379-385,
XP028336715, ISSN: 0924-4247, DOI:
10.1016/J.SNA.2011.09.027 [extrait le 2011-10-10]**
• **LAROCHE M ET AL: "HIGHLY DIRECTIONAL
RADIATION GENERATED BY A TUNGSTEN
THERMAL SOURCE", OPTICS LETTERS,
OPTICAL SOCIETY OF AMERICA, US, vol. 30, no.
19, 1 octobre 2005 (2005-10-01), pages 2623-2625,
XP001235370, ISSN: 0146-9592, DOI:
10.1364/OL.30.002623**

**Description**

**DOMAINE TECHNIQUE**

L'invention concerne un dispositif émissif lumineux à structures diffractives dans lequel est codé un hologramme synthétique, formant un « hologramme émissif lumineux » obtenu via l'échauffement d'une couche métallique dans laquelle est codé l'hologramme synthétique. L'invention concerne également un capteur de gaz comportant un tel dispositif émissif lumineux, ainsi qu'un dispositif photovoltaïque comportant un tel dispositif émissif lumineux.

**ART ANTÉRIEUR**

Afin de pouvoir contrôler la qualité de l'air, il existe un besoin d'avoir des systèmes de détection fiables et peu coûteux permettant de mesurer de faibles concentrations de composés organiques volatiles, de monoxyde de carbone ou encore de dioxyde de carbone. La spectroscopie est une voie d'application privilégiée dans le domaine de la détection des gaz, qui utilise la signature spectrale des composés chimiques à détecter pour permettre la mesure de leurs concentrations. Par exemple, le spectre d'absorption du $CO_2$ comporte notamment un pic très prononcé autour de 4,25 $\mu$m (donc dans le domaine infrarouge). Un dispositif de détection de $CO_2$ par spectroscopie peut donc détecter la présence de ce gaz via la détection d'un tel pic d'absorption dans le spectre d'un signal optique se trouvant en présence de ce gaz. Son application dans des systèmes à bas coût reste toutefois problématique et nécessite la mise en oeuvre de composants efficaces en énergie et faciles à produire en masse.

Le principe de l'analyse spectroscopique repose sur la segmentation spectrale d'un signal optique. Cette segmentation est généralement réalisée par un élément dispersif tel qu'un réseau de diffraction, ou sélectif tel qu'un filtre chromatique.

Un capteur de gaz se basant sur le principe de l'analyse spectroscopique comporte donc des éléments réalisant une émission d'un signal optique, une mise en forme du signal optique, une zone d'interaction entre le gaz à détecter et le signal optique, une segmentation spectrale du signal optique qui a interagie avec le gaz, et une détection.

Dans le cas d'un capteur de gaz destiné à réaliser la détection d'un ou plusieurs gaz présentant des pics d'absorption dans le domaine infrarouge (IR), comme par exemple le $CO_2$, la génération d'un signal optique dans l'infrarouge peut être obtenue à moindre coût par l'utilisation d'un filament incandescent. Un filament incandescent émet un signal optique lié à la température du filament, ce phénomène étant décrit sous le terme d'émission de corps noir. Lorsque sa température augmente, un corps noir émet un signal optique plus intense orienté vers les basses longueurs d'ondes. La détection d'une bande spectrale autour de 4,25 $\mu$m nécessite donc un filament chauffé à une température la plus haute possible. Cette température élevée doit toutefois être compatible avec les contraintes d'alimentation et de vieillissement nécessaires au bon fonctionnement du capteur.

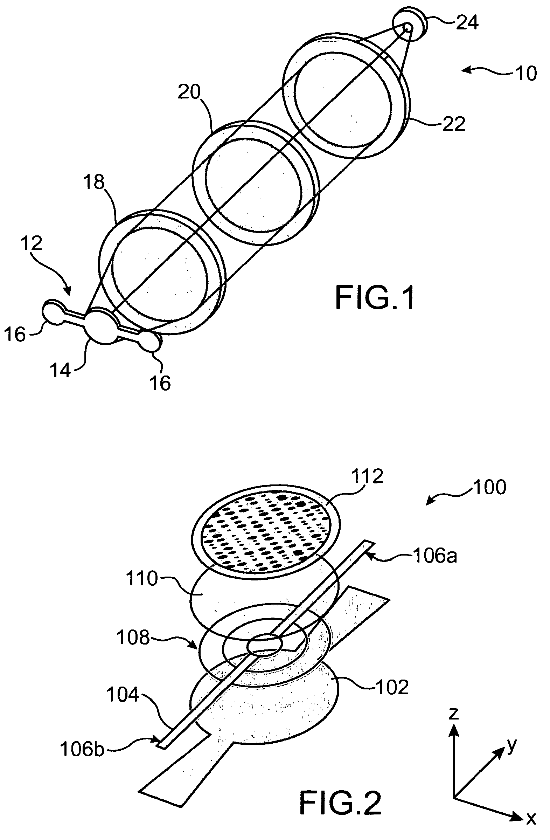

La figure 1 représente schématiquement un capteur de gaz 10 destiné à réaliser une détection de $CO_2$. Le capteur 10 comporte une source lumineuse infrarouge formée par un filament incandescent 12, par exemple composé de tungstène. Ce filament incandescent 12 comporte une plaque de radiation centrale 14 reliée à deux plots de connexion électrique 16. Une telle source infrarouge peut être réalisée par la mise en oeuvre de procédés collectifs de la microélectronique, comme décrit par exemple dans le document de P. Barritault et al., "Mid-IR source based on a free-standing microhotplate for autonomous CO2 sensing in indoor applications", Sensors and Actuators A: Physical, Volume 172, Issue 2, December 2011, pages 379-385. Le filament incandescent 12 émet une radiation suivant la théorie du corps noir lorsqu'il est chauffé à haute température, par exemple à 700°C. L'émission lumineuse infrarouge obtenue est mise en forme par une première optique 18 qui collimate le faisceau lumineux pour le diriger vers un filtre chromatique 20, puis une seconde optique 22 concentre le faisceau filtré sur un détecteur 24. Le filtre 20 permet la limitation du spectre détecté à une seule bande spectrale autour de la longueur d'onde à détecter, c'est-à-dire 4,25 $\mu$m pour le $CO_2$.

L'utilisation d'un filament incandescent comme source lumineuse du capteur a pour avantage d'être peu consommatrice en énergie et peut être fabriqué en grand nombre. Toutefois, un tel capteur 10 nécessite l'utilisation de plusieurs optiques de mise en forme du faisceau lumineux et d'un filtre de limitation spectrale du fait que le rayonnement lumineux du filament incandescent 12 est à spectre large et isotrope (l'émission ne privilégie aucune direction particulière).

Il existe des solutions permettant d'exacerber une forme de sélectivité du rayonnement émis par une source lumineuse. Cette sélectivité met généralement en oeuvre des effets liés à la présence de polaritons qui sont des éléments de couplage entre des vibrations de surface de la matière chauffée et des photons. La présence des polaritons sur une surface de matériau ne se traduit pas par une émission de lumière car l'énergie reste localisée à la surface du matériau, au niveau des modes de surface. Pour obtenir une émission de lumière, un artifice de résonance doit être utilisé, comme par exemple un prisme ou un réseau. Le vecteur d'onde de la lumière peut alors sortir du plan de la surface de la matière chauffée.

Le document de F. Marquier et al., "Coherent spontaneous emission of light by thermal sources" ; Physical Review B, vol. 69, Issue 15, id. 155412 (2004), décrit une source lumineuse infrarouge comportant un réseau périodique réalisé dans une couche de matériau. Ce matériau est chauffé et irradie suivant le spectre du corps noir formé par ce matériau. Le matériau choisi présente la propriété de permettre la propagation de SPP ou « Surface Phonon Polaritons » à son interface avec l'air (matériau polaire). Lorsque ces ondes guidées rencontrent le réseau périodique, un phénomène de couplage se produit et de l'énergie est transférée sous la forme d'une onde lumineuse se propageant dans l'air de manière cohérente dans une certaine gamme de longueurs d'ondes.

Deux régimes particuliers d'émission ont été mis en évidence, en fonction du pas du réseau. Pour un pas de 3 $\mu$m, la relation de dispersion est asymptotique dans la zone de propagation libre. Le couplage ne peut alors se faire que vers un ensemble restreint de longueurs d'ondes mais pour une gamme importante de vecteurs d'onde. L'émission obtenue avec un tel pas de 3 $\mu$m est monochromatique et isotrope. Pour un pas de 6,25 $\mu$m, la relation de dispersion relie chaque longueur d'onde de propagation libre à un vecteur d'onde spécifique. L'émission lumineuse se fait dans ce cas suivant un large spectre, chaque longueur d'onde étant émise suivant une direction angulaire précise.

L'aspect directionnel de l'émission est associé à la présence d'un certain degré de cohérence spatiale de l'émission de lumière. Une longueur de cohérence spatiale est ainsi estimée de l'ordre de quelques centaines de microns.

Dans le cas d'une couche de SiC, le phénomène de couplage est limité à une gamme spectrale comprise entre 10 $\mu$m et 13 $\mu$m, gamme pour laquelle le matériau a un indice complexe non nul. Pour étendre l'application au domaine de détection du $CO_2$, la couche de matériau peut être réalisée en tungstène qui peut être chauffé à une température de plus de 2000 K, comme décrit dans le document de M. Laroche et al., "Highly directional radiation generated by a tungsten thermal source", Optics Letters, Vol. 30, Issue 19, pp. 2623-2625 (2005). Avec un réseau périodique présentant un pas de 3 $\mu$m et une profondeur de gravure de 150 nm, il est possible d'émettre alors un faisceau directionnel à une longueur d'onde de 4,25 $\mu$m avec un angle d'émission d'environ 25°.

Le document de I. Puscasu et al., "Photonic crystals enable infrared gas sensors", Nanoengineering: Fabrication, Properties, Optics, and Devices. Edited by Dobisz, Elizabeth A.; Eldada, Louay A. Proceedings of the SPIE, Volume 5515, pp. 58-66 (2004), décrit des phénomènes similaires obtenus par l'interaction de plasmons (modes de surface à des interfaces métalliques) dans des structures de type cristaux photoniques. La source lumineuse comporte ici une couche métallique comprenant une structuration réalisée en 2 dimensions sous la forme d'une matrice de trous profonds de quelques microns. La couche métallique est disposée sur une membrane en semi-conducteur (silicium). Le réseau de trous traverse la couche métallique et structure également la membrane semi-conductrice. La présence de cette structure décrite comme un cristal photonique a pour effet de renforcer l'émission dans une bande étroite de longueurs d'ondes correspondant à la bande spectrale d'absorption du $CO_2$. La position spectrale de la bande d'émission peut être accordée par le pas du réseau de trous. Toutefois, avec un tel dispositif, l'énergie perdue en dehors de cette bande n'est pas transférée dans la bande d'émission : le niveau du pic d'énergie reste inférieur à celui du corps noir porté à la même température.

## EXPOSÉ DE L'INVENTION

Un but de la présente invention est de proposer un nouveau type de dispositif émissif lumineux permettant de contrôler la bande spectrale d'émission, la direction angulaire d'émission ainsi que la forme du rayonnement lumineux émis par le dispositif.

Pour cela, il est proposé un dispositif émissif lumineux comportant au moins :

- une couche métallique apte à s'échauffer et à propager des ondes de surface consécutives à l'échauffement de la couche métallique, la couche métallique étant structurée telle qu'elle comporte plusieurs motifs de diffraction aptes à réaliser une diffraction des ondes de surface vers des modes de propagation en espace libre, et dans laquelle un hologramme synthétique apte à contrôler la forme d'un rayonnement lumineux destiné à être émis par le dispositif émissif lumineux via le contrôle d'un front d'onde destiné à être émis est codé tel que l'hologramme synthétique correspond à une image de phase codée en chaque pixel par un décalage de position d'un des motifs de diffraction par rapport à une position de référence dudit un des motifs de diffraction;
- des moyens aptes à chauffer la couche métallique.

Un tel dispositif émissif lumineux permet donc d'optimiser son émission lumineuse en y introduisant, via les motifs de diffraction, une perturbation périodique génératrice de couplage entre des modes guidés confinés en surface de la couche métallique (ondes de surface) et des modes de propagation en espace libre. De plus, un tel dispositif émissif lumineux comporte également un hologramme synthétique modifiant la phase de l'onde diffractée grâce une distribution calculée des motifs de diffraction pour chaque pixel, cet hologramme synthétique permettant notamment de contrôler la forme du rayonnement lumineux émis par le dispositif via le contrôle du front d'onde émis.

L'adaptation du front d'onde réalisée par l'hologramme synthétique permet donc d'obtenir une distribution d'intensité choisie dans le plan de reconstruction image, c'est-à-dire dans le plan destiné à recevoir l'émission lumineuse du dispositif. Ce plan répond au critère de l'optique de Fourier pour la lecture de l'hologramme synthétique, ou hologramme à transformée de Fourier.

La couche de matériau métallique peut correspondre ici à une couche de matériau polaire dans laquelle est codé l'hologramme synthétique.

Dans chaque pixel, l'image de phase peut être codée par un décalage, le long d'un premier axe, d'une position dudit un des motifs de diffraction par rapport à la position de référence dudit un des motifs de diffraction, les positions de référence des motifs de diffraction pouvant être définies comme correspondant à des intersections d'une grille de n lignes et de m colonnes régulièrement espacées les unes des autres, le premier axe pouvant être parallèle aux n lignes ou aux m colonnes de la grille, les motifs de diffraction pouvant être régulièrement espacés les uns des autres le long d'un deuxième axe perpendiculaire au premier axe, et chaque décalage de position d'un des motifs de diffraction par rapport à sa position de référence peut être inférieur à une distance p séparant deux lignes ou deux colonnes adjacentes de la grille, avec n et m nombres entiers supérieurs à 1.

Chaque motif de diffraction est dans ce cas décalé de sa position nominale d'une valeur correspondant au décalage de phase souhaité sur le front d'onde à générer par le dispositif. Les décalages de position d'au moins une partie des motifs de diffraction sont dans ce cas non nuls.

Dans ce cas, les motifs de diffraction peuvent avoir des dimensions sensiblement similaires les uns par rapport aux autres, et/ou un rapport entre une dimension de chaque motif de diffraction selon le deuxième axe et une dimension dudit chaque motif de diffraction selon le premier axe peut être supérieur ou égal à environ 1,5, et/ou une surface d'une section de chaque motif de diffraction, dans un plan principal de la couche métallique, peut être supérieure ou égale environ $p^2/2$, et/ou les motifs de diffraction peuvent comporter des ouvertures réalisées à travers au moins une partie de l'épaisseur de la couche métallique et une surface d'une section d'une ou des ouvertures de chaque motif de diffraction, dans un plan principal de la couche métallique, peut être supérieure ou égale à environ $p^2/2$.

Selon une variante, chaque motif de diffraction peut former une partie d'un réseau de diffraction comprenant plusieurs fentes parallèles les unes par rapport aux autres, et, dans chaque pixel, l'image de phase peut être codée par un décalage, le long d'un premier axe, d'une position des parties des fentes dudit un des motifs de diffraction par rapport à la position de référence dudit un des motifs de diffraction, les positions de référence des motifs de diffraction pouvant être définies comme correspondant aux positions dans lesquelles les parties des fentes de tous les motifs de diffraction sont alignées les unes par rapport aux autres.

Les décalages de position des motifs de diffraction correspondent dans ce cas à des décalages de phase du réseau de diffraction introduits individuellement pour chaque pixel de l'hologramme synthétique.

Les décalages de position d'au moins une partie des motifs de diffraction sont dans ce cas non nuls.

Les motifs de diffraction peuvent comporter des ouvertures réalisées à travers au moins une partie de l'épaisseur de la couche métallique.

Chacune des ouvertures peut être de forme sensiblement elliptique ou chacune des ouvertures peut correspondre à un segment de ligne.

La couche métallique peut comporter une épaisseur supérieure à environ 200 nm, et les ouvertures peuvent être réalisées dans une partie de la couche métallique de profondeur comprise entre environ 50 nm et 200 nm.

Les moyens aptes à chauffer la couche métallique peuvent comporter au moins un élément électriquement conducteur couplé thermiquement avec la couche métallique et apte à s'échauffer lorsqu'il est traversé par un courant électrique.

L'élément électriquement conducteur peut comporter un empilement de type TiN/Pt/TiN, et/ou la couche métallique peut comporter du tungstène et/ou du platine. Le métal de la couche métallique peut être choisi en fonction de la gamme des longueurs d'ondes destinées à être diffractées et émises sous la forme d'un hologramme par le dispositif. Ainsi, une couche métallique à base de SiC peut être adaptée pour une émission dans une bande de longueurs d'ondes comprises entre environ 10,5 $\mu$m et 12,5 $\mu$m. Une couche métallique à base de tungstène et/ou de platine a toutefois pour avantage, par rapport au SiC, de permettre une émission dans une plus large gamme de longueurs d'ondes, et notamment dans une gamme incluant le pic d'absorption du $CO_2$.

Le dispositif émissif lumineux peut comporter en outre un élément de support mécanique sur lequel est disposée la couche métallique et/ou, lorsque les moyens aptes à chauffer la couche métallique comportent l'élément électriquement conducteur, un élément isolant électrique disposé entre l'élément électriquement conducteur et la couche métallique.

Les moyens aptes à chauffer la couche métallique peuvent comporter au moins un dispositif apte à faire circuler un courant électrique à travers la couche métallique ou, lorsque lesdits moyens comportent l'élément électriquement conducteur, un dispositif apte à faire circuler un courant électrique à travers l'élément électriquement conducteur.

Le dispositif émissif lumineux peut comporter en outre au moins une lentille de Fourier disposée à une distance focale objet de la couche métallique et à une distance focale image d'un plan destiné à recevoir l'émission lumineuse du dispositif. Une telle lentille de Fourier permet de reconstruire l'image de l'hologramme au niveau du plan destiné à recevoir l'émission lumineuse du dispositif.

En variante, une fonction de phase de l'hologramme synthétique codé dans la couche métallique peut comporter une fonction optique sensiblement similaire à une fonction de convergence d'une lentille de Fourier.

Ainsi, la reconstruction de l'image de l'hologramme est obtenue dans son plan de reconstruction sans faire appel à une lentille de Fourrier, ce qui permet de réaliser une lecture directe du front d'onde émis.

Une fonction de phase de l'hologramme synthétique codé dans la couche métallique peut comporter une fonction optique, par exemple de type lentille cylindrique, apte à compenser une déformation du dispositif émissif lumineux lors de l'échauffement de la couche métallique. Ainsi, une déformation due à l'échauffement de la couche métallique peut être compensée en prenant en compte cette déformation lors du codage de l'hologramme synthétique, et en intégrant par exemple une déformation inverse, de type lentille cylindrique, dans l'hologramme qui compense celle subie par le dispositif émissif lumineux.

Une image de l'hologramme synthétique peut être une fente. De manière générale, l'image de l'hologramme synthétique est choisie en fonction de la forme du rayonnement lumineux souhaité, et donc de l'application envisagée du dispositif émissif lumineux. Une image correspondant à une fente est bien adaptée lorsque le dispositif émissif lumineux est destiné à faire partie d'un capteur de gaz.

L'invention concerne également un capteur de gaz comportant au moins un dispositif émissif lumineux selon l'invention et un détecteur apte à détecter la présence d'au moins un gaz dans un espace traversé par au moins une image de l'hologramme émise par le dispositif émissif lumineux. Par rapport aux capteurs de gaz de l'art antérieur, un tel capteur de gaz nécessite moins d'éléments, et notamment moins d'éléments optiques de mise en forme du rayonnement lumineux émis par la source lumineuse incandescente.

L'image codée dans l'hologramme peut notamment être adaptée à la géométrie du détecteur du capteur de gaz.

Le détecteur peut être apte à réaliser une détection spectrale de l'image de l'hologramme au moins dans une gamme de longueurs d'ondes comportant au moins une bande d'absorption spectrale du ou des gaz destiné(s) à être détecté(s) par le capteur.

Dans ce cas, le détecteur peut être apte à réaliser une détection spectrale de plusieurs images de l'hologramme émis par le dispositif émissif lumineux dans plusieurs gammes de longueurs d'ondes dont l'une au moins comporte la bande d'absorption spectrale du ou des gaz destiné(s) à être détecté(s) par le capteur.

En variante, le détecteur peut être apte à réaliser une détection spectrale d'une seule image de l'hologramme émis par le dispositif émissif lumineux dans une seule gamme de longueurs d'ondes comportant la bande d'absorption spectrale du gaz destiné à être détecté par le capteur.

Dans une autre variante, le détecteur peut comporter au moins :

- un élément réfléchissant disposé en regard du dispositif émissif lumineux et apte à refléter l'image de l'hologramme ayant traversé l'espace comprenant le ou les gaz vers le dispositif émissif lumineux ;
- un dispositif de mesure de la résistance électrique de la couche métallique ou, lorsque les moyens aptes à chauffer la couche métallique comportent l'élément électriquement conducteur, un dispositif de mesure de la résistance électrique de l'élément électriquement conducteur.

Dans ce cas, seul l'élément réfléchissant, et non toute une structure détectrice, est destiné à se trouver dans le plan de reconstruction de l'image de l'hologramme, ce qui simplifie la réalisation du capteur. La détection peut dans ce cas être obtenue par la mesure de variation de la valeur de résistance électrique de la source lumineuse, donc de la couche métallique ou de l'élément électriquement conducteur du dispositif émissif lumineux.

L'invention concerne également un dispositif photovoltaïque comportant au moins un dispositif émissif lumineux, le dispositif émissif lumineux comportant au moins :

- une couche métallique apte à s'échauffer et à propager des ondes de surface consécutives à l'échauffement de la couche métallique, la couche métallique étant structurée telle qu'elle comporte plusieurs motifs de diffraction aptes à réaliser une diffraction des ondes de surface vers des modes de propagation en espace libre, et dans laquelle un hologramme synthétique apte à contrôler la forme d'un rayonnement lumineux destiné à être émis par le dispositif émissif lumineux via le contrôle d'un front d'onde destiné à être émis est codé tel que l'hologramme synthétique correspond à une image de phase codée en chaque pixel par un décalage de position d'un des motifs de diffraction par rapport à une position de référence dudit un des motifs de diffraction ;

et dans lequel la couche métallique du dispositif émissif lumineux est apte à recevoir des rayons lumineux reçus par le dispositif photovoltaïque, et comportant en outre au moins un élément de conversion photovoltaïque apte à recevoir une image de l'hologramme destinée à être émise par le dispositif émissif lumineux.

Ainsi, le dispositif émissif lumineux, qui est apte à absorber le large spectre solaire reçu par le dispositif photovoltaïque, permet de réémettre l'énergie solaire reçue vers l'élément de conversion photovoltaïque dans une gamme spectrale adaptée à cet élément, et ainsi augmenter le rendement de conversion de l'élément par rapport à un dispositif photovoltaïque comportant le même élément de conversion photovoltaïque mais ne comportant pas le dispositif émissif lumineux selon l'invention.

## BRÈVE DESCRIPTION DES DESSINS

La présente invention sera mieux comprise à la lecture de la description d'exemples de réalisation donnés à titre purement indicatif et nullement limitatif en faisant référence aux dessins annexés sur lesquels :

- la figure 1 représente schématiquement un capteur de gaz selon l'art antérieur;
- les figures 2 et 3 représentent schématiquement un dispositif émissif lumineux, objet de la présente invention, selon un mode de réalisation particulier ;
- la figure 4 représente le principe de l'hologramme synthétique codé dans le dispositif émissif lumineux, objet de la présente invention ;
- les figures 5 et 6 représentent des exemples de motifs de diffraction réalisés dans une couche métallique d'un dispositif émissif lumineux, objet de la présente l'invention ;
- la figure 7 représente un exemple de dispositions de motifs de diffraction par rapport à leur position de référence, ces motifs codant un hologramme synthétique ;
- la figure 8 représente un schéma de lecture d'un hologramme synthétique ;
- les figures 9 et 10 représentent des méthodes de reconstruction d'image d'un hologramme à partir d'un front d'onde modifié ;
- la figure 11 représente des courbes d'émissivité angulaire obtenues pour différents matériaux et différents motifs de diffraction formés dans le dispositif émissif lumineux, objet de la présente invention ;
- les figures 12 à 16 représentent un capteur de gaz, objet de la présente invention, selon plusieurs modes de réalisation ;
- la figure 17 représente un dispositif photovoltaïque, objet de la présente invention, selon un mode de réalisation particulier.

Des parties identiques, similaires ou équivalentes des différentes figures décrites ci-après portent les mêmes références numériques de façon à faciliter le passage d'une figure à l'autre.

Les différentes parties représentées sur les figures ne le sont pas nécessairement selon une échelle uniforme, pour rendre les figures plus lisibles.

Les différentes possibilités (variantes et modes de réalisation) doivent être comprises comme n'étant pas exclusives les unes des autres et peuvent se combiner entre elles.

## EXPOSÉ DÉTAILLÉ DE MODES DE RÉALISATION PARTICULIERS

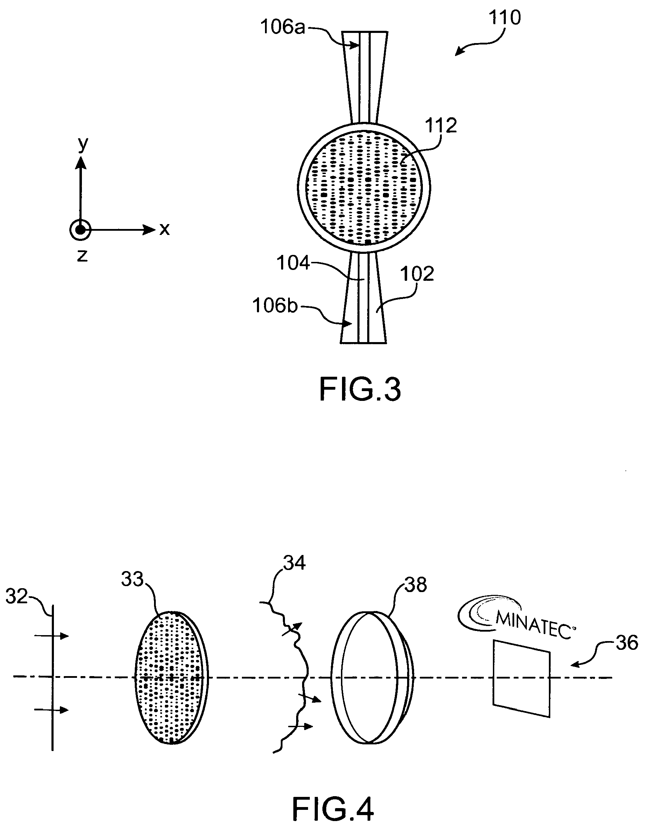

Un dispositif émissif lumineux 100 selon un mode de réalisation particulier est représenté schématiquement sur les figures 2 et 3 (en vue éclatée en 3 dimensions sur la figure 2 et en vue de dessus sur la figure 3).

Le dispositif émissif lumineux 100 comporte un élément de support mécanique 102 par exemple réalisé à base de $Si_3N_4$. Un élément électriquement conducteur 104, correspondant par exemple à un empilement TiN/Pt/TiN, est réalisé sur l'élément de support mécanique 102. L'élément électriquement conducteur 104 est apte à s'échauffer lorsqu'il est traversé par un courant électrique. Deux parties longitudinales 106a et 106b de l'élément électriquement conducteur 104 (qui reposent sur deux parties longitudinales de l'élément de support mécanique 102) forment deux plots de connexion électrique de l'élément électriquement conducteur 104, et donc du dispositif 100.

L'élément électriquement conducteur 104 comporte également une partie centrale 108, formée de plusieurs portions de matériau électriquement conducteur en forme d'anneaux concentriques et reliées électriquement aux deux parties longitudinales 106a, 106b. Cette partie centrale 108 de l'élément électriquement conducteur 104 (qui repose sur une partie centrale en forme de disque de l'élément de support mécanique 102) est destinée à générer l'échauffement. Le dispositif 100 comporte également un élément isolant électrique 110, ici en forme de disque, comprenant du $SiO_2$ et recouvrant la partie centrale 108 de l'élément électriquement conducteur 104.

Le dispositif émissif lumineux 100 comporte en outre un élément complémentaire de diffusion thermique correspondant à une couche métallique 112, ici en forme de disque, disposée en regard de la partie centrale 108 de l'élément électriquement conducteur 104 et comprenant par exemple du tungstène et/ou du platine, structurée par un réseau d'ouvertures (c'est-à-dire de creux ou de trous) formant des éléments de diffraction, ou motifs de diffraction. De plus, ce réseau d'ouvertures formé dans la couche 112 est également réalisé tel qu'il code en son sein un hologramme synthétique. Les motifs de la structuration réalisée dans la couche 112 peuvent être réalisés, par exemple par gravure, à travers toute l'épaisseur de la couche 112 ou seulement une partie de l'épaisseur de la couche 112, cette partie étant par exemple comprise entre environ 50 nm et 200 nm.

La couche métallique 112 est isolée électriquement de l'élément électriquement conducteur 104 grâce à l'élément isolant électrique 110 disposé entre la couche métallique 112 et l'élément électriquement conducteur 104. Par contre, la couche métallique 112 est couplée thermiquement avec la partie centrale 108 de l'élément électriquement conducteur 104. Ainsi, l'échauffement de la partie centrale 108 de l'élément électriquement conducteur 104 produit par le passage du courant à travers celui-ci est transmis à la couche métallique 112. L'échauffement de la couche métallique 112 produit alors des ondes se propageant en surface de la couche métallique 112. Ces ondes de surface sont diffractées par les motifs de diffraction réalisés dans la couche métallique 112 vers des modes de propagation libre, l'émission lumineuse obtenue se faisant selon l'hologramme synthétique codé dans la couche métallique 112.

Le principe d'un hologramme synthétique tel que celui codé dans la couche 112 est schématiquement représenté en figure 4. Un front d'onde 32 arrivant de manière incidente sur une couche 33 dans laquelle est codé l'hologramme synthétique est modifié par les structurations de la couche 33 (correspondant aux motifs de diffraction dans le cas présent), générant un front d'onde modifié 34 transportant l'information relative à l'hologramme codé. L'image désirée 36 est obtenue par reconstruction en faisant traverser le front d'onde modifié 34 à travers une lentille de Fourier 38 réalisant une transformée de Fourier inverse du front d'onde modifié 34.

L'image et le front d'onde modifié sont reliés par une transformation de Fourier. La conception de l'hologramme revient à définir une structure permettant de générer le front d'onde modifié 34. Pour cela, cette structure doit pouvoir modifier de façon contrôlée la phase de l'onde incidente. Une telle structure peut correspondre à une structure de diffraction fabriquée dans une couche métallique (hologramme d'amplitude).

Dans le dispositif émissif lumineux 100, la couche métallique 112 n'est pas traversée par un front d'onde arrivant de manière incidente sur celle-ci. En effet, dans le dispositif émissif lumineux 100, c'est l'échauffement de la couche métallique 112 qui provoque la génération d'ondes de surface qui sont ensuite diffractées par les motifs de diffraction vers des modes de propagation en espace libre, générant ainsi l'émission lumineuse d'un front d'onde similaire au front d'onde 34, transportant l'information relative à l'hologramme codé.

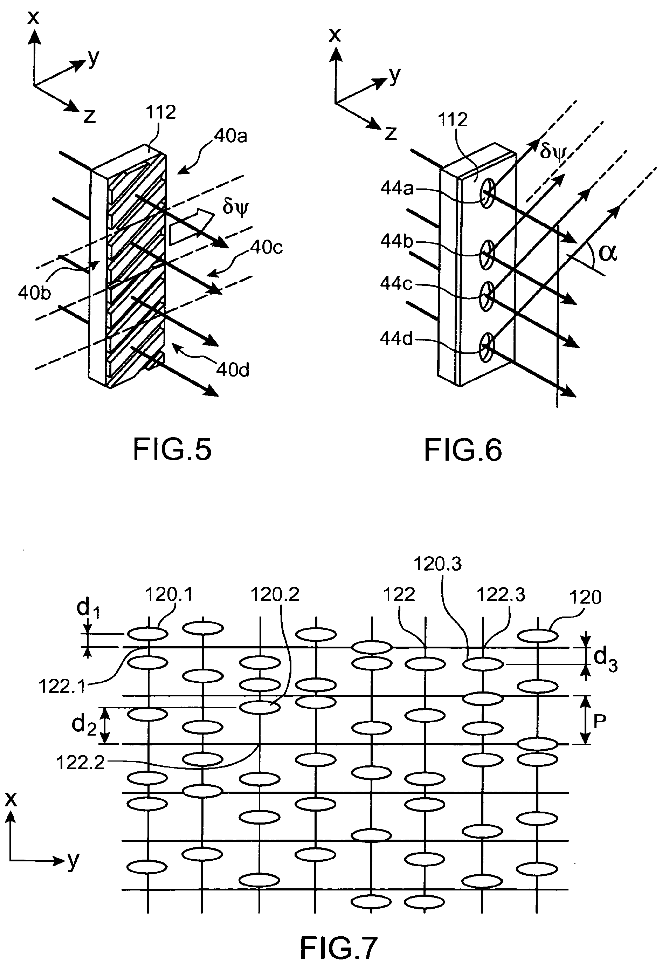

Un premier exemple de réalisation des motifs de diffraction réalisés dans la couche métallique 112 est représenté sur la figure 5. La couche métallique 112 est structurée selon un motif formant un réseau de diffraction. La modification de la phase des ondes générées en surface de la couche métallique 112 est obtenue en modifiant la phase du réseau de diffraction au niveau de chaque pixel de l'image de l'hologramme, c'est-à-dire au niveau de chaque motif de diffraction. Sur l'exemple de la figure 5, quatre parties de la couche métallique 112 correspondent à quatre pixels de l'hologramme et forment quatre motifs de diffraction 40a - 40d. Le réseau de diffraction formé par les motifs de diffraction correspondant aux pixels de l'hologramme est formé par un réseau de fentes orientées parallèlement les unes par rapport aux autres. Chaque motif de diffraction est donc formé par une partie de ces fentes localisée au niveau de chaque partie de la couche métallique 112 associée au pixel. Sur la figure 5, les fentes sont orientées selon une direction incluse dans le plan (X,Y). Pour chaque pixel, le décalage de phase réalisé correspond à un décalage de la partie des fentes correspondante le long de l'axe Y par rapport à une position de référence. Les positions de référence des motifs de diffraction correspondent aux positions dans lesquelles les parties des fentes de tous les motifs de diffraction sont alignées les unes par rapport aux autres. Le réseau de diffraction représente l'image binaire des interférences entre les ondes de surface générées par l'échauffement de la couche métallique 112 et le front d'onde émis par la couche métallique 112 correspondant à l'hologramme. A la reconstruction de l'hologramme, le front d'onde émis par la couche métallique 112 est récupéré suivant les ordres de diffraction du réseau, l'ordre 0 ne comportant pas de modification de sa phase. Le document "Binary computer-generated holograms" de Wai-Hon Lee, Applied Optics, vol.18, n°21, 1er novembre 1979, décrit par exemple la réalisation d'un tel hologramme synthétique.

Un deuxième exemple de réalisation des motifs de diffraction réalisés dans la couche métallique 112 est représenté sur la figure 6. La couche métallique 112 est structurée selon un maillage de motifs de diffraction. Sur la figure 6, quatre motifs de diffraction 44a -44d, permettant de réaliser la diffraction des ondes de surface au niveau de quatre pixels sont représentés et ont chacun, dans le plan (X,Y) de la couche métallique 112, une section de forme ovale. L'introduction de décalages dans la répartition des motifs selon un des axes du plan de la couche métallique 112 (l'axe X sur l'exemple de la figure 6), dans chaque partie de la couche métallique 112 correspondant à un pixel, revient à introduire localement des déphasages sur les ondes générées suivant certains ordres de diffraction du réseau. Un hologramme obtenu avec une telle structure de diffraction est appelé hologramme à détour de phase et est par exemple décrit dans le document de A.W. Lohmann and D.P. Paris (1967), "Binary Fraunhofer Holograms, Generated by Computer," Appl. Opt. 6, 1739-1748. Une telle structure de diffraction est plus simple à réaliser que celle décrite en liaison avec la figure 5 puisqu'elle se compose de la répétition d'un motif de taille voisine du pas de la structure de diffraction et non de la répétition de lignes de largeur bien inférieure au pas de la structure de diffraction.

Ainsi, dans la couche métallique 112, un hologramme synthétique est codé tel qu'une image de phase de chaque pixel de l'hologramme soit codée par un décalage, le long d'un premier axe, d'une position d'un des motifs de diffraction par rapport à une position de référence de ce motif de diffraction. La figure 7 représente schématiquement un exemple de disposition des motifs de diffraction dans une partie de la couche 112 lorsque les motifs de diffraction sont réalisés par des ouvertures telles que précédemment décrites en liaison avec la figure 6. Dans cet exemple de réalisation, les motifs de diffraction correspondent à des ouvertures 120 réalisées à travers une partie de l'épaisseur de la couche 112. Les positions de référence 122, ou positions nominales, des motifs de diffraction 120 sont définies comme correspondant à des intersections d'une grille de n lignes et de m colonnes régulièrement espacées les unes des autres.

Les nombres n et m sont des entiers dont les valeurs correspondent aux nombres de lignes et de colonnes de pixels de l'hologramme. Le premier axe est ici parallèle aux m colonnes de la grille. Les motifs de diffraction sont régulièrement espacés les uns des autres le long d'un deuxième axe (axe Y) perpendiculaire au premier axe, et chaque décalage de position d'un des motifs de diffraction par rapport à la position de référence est inférieur à une distance p séparant deux lignes ou deux colonnes adjacentes de la grille. Plusieurs exemples de décalages d1, d2 et d3 de motifs de diffraction 120.1, 120.2 et 120.3 par rapport à leur position de référence 122.1, 122.2 et 122.3 sont représentés sur la figure 7.

Il convient de noter que le codage de l'hologramme peut entraîner un décalage nul pour certains motifs de diffraction par rapport à leur position de référence.

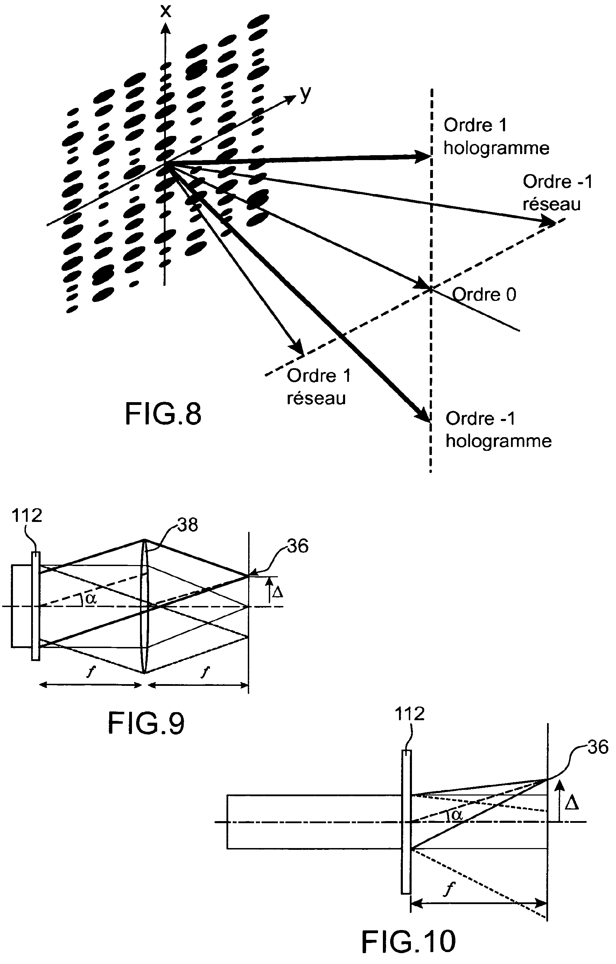

La figure 8 représente un schéma de lecture d'un hologramme à détour de phase, similaire à celui codé dans la couche métallique 112 et tel que précédemment décrit en liaison avec les figures 6 et 7. Seule la diffraction selon l'axe de modification de la position des motifs (axe X dans l'exemple décrit ici) permet de générer le front d'onde émis depuis la couche métallique 112. Ce front d'onde se retrouve suivant les ordres de diffraction conjugués positifs et négatifs de l'hologramme, et principalement suivant les ordres conjugués 1 et -1 de l'hologramme.

La méthode de reconstruction de l'image à partir du front d'onde émis peut être de deux types.

Dans un premier cas représenté schématiquement sur la figure 9, la lentille de Fourier 38 est positionnée à distance focale objet de la couche métallique 112 dans laquelle est codé l'hologramme. L'image 36 est reconstruite dans un plan de reconstruction se trouvant à une distance focale image de la lentille 38. La taille de l'image et son décalage par rapport à l'axe optique sont donnés par un paramètre $\Delta$ calculé à partir du pas du réseau de motifs de diffraction $\Lambda$, de la longueur d'onde $\lambda$ et de la focale $f$ de la lentille 38 tel que :

$$\Delta = \frac{\lambda f}{\Lambda} \qquad (1)$$

Dans un deuxième cas représenté sur la figure 10, la fonction de convergence peut être introduite dans la fonction de phase de l'hologramme, les motifs de la structuration de la couche métallique 112 étant réalisés tels que la couche métallique 112 réalise cette fonction de convergence. La phase de l'hologramme $\Delta\varphi(r)$ est modifiée radialement suivant la valeur de focale souhaitée telle que :

$$\Delta\varphi(r) = \frac{\pi \times r^2}{\lambda f} \qquad (2)$$

Le codage de la fonction de phase de la lentille de Fourier dans la couche métallique 112 est limité par la taille de la cellule hologramme. On peut définir une limite lorsque la variation de phase de la lentille de Fourier à l'échelle d'une cellule de l'hologramme dépasse $2\pi$. On en déduit un rayon limite $R_{lim}$, et une focale limite $f_{lim}$, tels que :

$$R_{lim} = \frac{\lambda f + \Lambda^2}{2\Lambda} \approx \frac{\lambda f}{2\Lambda} \qquad (3)$$

$$f_{lim} = \frac{2\Lambda \times R_{max} - \Lambda^2}{\lambda} \approx 2\frac{\Lambda \times R_{max}}{\lambda} \qquad (4)$$

A titre d'exemple, à une longueur d'onde $\lambda$ de 4,25 $\mu$m et pour un pas d'hologramme $\Lambda$ de 5 $\mu$m la focale limite $f_{lim}$ pour un rayon R de 250 $\mu$m est de 0,6 mm.

Le deuxième cas de lecture exposé ci-dessus diffère du premier cas car l'image n'est visible que dans l'ordre 1 de l'hologramme, l'ordre conjugué -1 représente une fonction lentille de focale inversée, soit un faisceau divergent.

Le pas du réseau de motifs de diffraction $\Lambda$, ou pas d'hologramme, correspond à la distance séparant deux positions de référence de deux motifs de diffraction adjacents réalisés dans la couche métallique 112.

Etant donné l'hologramme codé dans la couche métallique 112, le dispositif émissif lumineux 100 forme un « hologramme émissif ». Chaque motif de diffraction réalisé dans la couche métallique 112 forme donc une source lumineuse ponctuelle non reliée à une onde externe au dispositif 100. L'émission de ces différentes sources est corrélée via un effet de cohérence spatiale dû à la propagation des ondes de surface dans la couche métallique 112 générées par l'échauffement de cette couche provoqué par le passage du courant dans l'élément électriquement conducteur 104.

Comme dans les dispositifs émissifs comportant un réseau périodique ou une structure de type cristal photonique, la génération d'onde de surface dans la couche métallique 112 est perturbée par la présence de la structuration périodique réalisée sur cette même surface. Du fait que les paramètres de la structuration périodique de la couche métallique 112 sont adaptés à la relation de dispersion des modes de surface, un couplage de ces modes vers des ondes propagatrices en espace libre est possible. De plus, conformément au principe des hologrammes à détour de phase, du fait que la répartition périodique de la structure de couplage est modifiée, le front d'onde de l'onde propagatrice est également modifié. Ainsi, dans le dispositif émissif 100, la génération d'un front d'onde contrôlé est obtenue à partir d'un phénomène d'incandescence. Ainsi, le dispositif 100 émet un faisceau lumineux dans une gamme de longueurs d'ondes et selon un angle d'émission α déterminés via le pas du réseau de motifs de diffraction formés dans la couche métallique 112, le faisceau lumineux émis par le dispositif 100 étant en outre distribué selon une image dont la forme et le positionnement correspondent à l'hologramme codé dans ce réseau de motifs de diffraction formés dans la couche métallique 112.

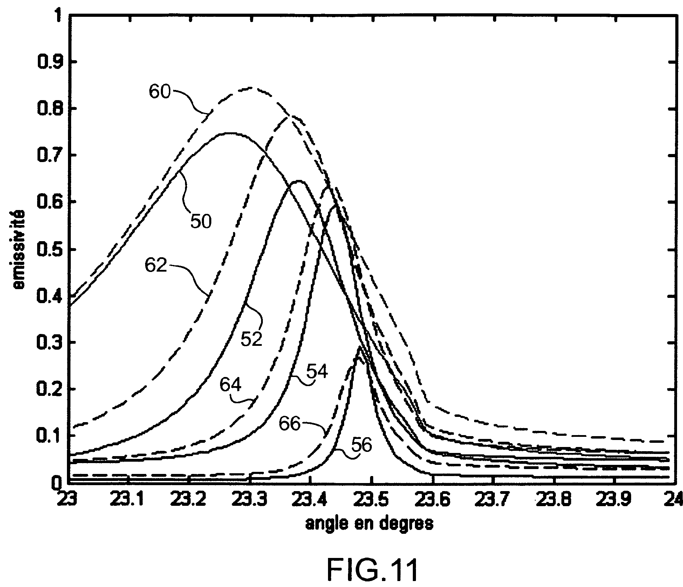

Les courbes de la figure 11 représentent l'émissivité angulaire de la couche métallique 112, c'est-à-dire le niveau d'émissivité obtenu en fonction de l'angle, dans laquelle un hologramme synthétique est codé par des ouvertures telles que celles précédemment décrites en liaison avec les figures 6-8, pour différentes profondeurs des ouvertures formant les motifs de diffraction et pour différents matériaux. Pour toutes ces courbes, l'émissivité est mesurée pour une longueur d'onde de 4,2 μm et pour un pas de réseau (distance entre les positions de référence de deux motifs de diffraction adjacents) égal à 3 μm. Les courbes 50, 52, 54 et 56 correspondent à l'émissivité obtenue avec une couche métallique 112 en tungstène, avec des ouvertures réalisées respectivement à des profondeurs de 50 nm, 100 nm, 150 nm et 200 nm. Les courbes 60, 62, 64 et 66 correspondent à l'émissivité obtenue avec une couche métallique 112 en platine, avec des ouvertures réalisées respectivement à des profondeurs de 50 nm, 100 nm, 150 nm et 200 nm. On voit sur ces courbes qu'un optimum est obtenu avec des ouvertures réalisées avec une profondeur se situant entre environ 100 nm et 150 nm.

Avantageusement, l'hologramme est calculé par une méthode de brouillage de la phase, comme par exemple décrit dans le document "Use of a random phase mask for the recording of Fourier transform holograms of data masks" de C.B. Burckhardt, Appl. Opt. 9(3), 695-700 (1970), et l'amplitude de l'hologramme est fixée de sorte que le taux de remplissage des cellules de l'hologramme, correspondant au rapport de la surface du motif de diffraction sur la surface de la partie de la couche métallique 112 correspondant à un pixel soit égal à au moins 50 %. De plus, le rapport entre la taille des motifs de la structuration de la couche métallique 112 dans la direction perpendiculaire au codage de phase (axe Y sur l'exemple de la figure 7) et la taille dans la direction du codage de phase (axe X sur l'exemple de la figure 7) est de préférence au moins égal à 1,5. Les motifs de diffraction ont également de préférence des dimensions sensiblement similaires les uns par rapport aux autres.

L'image de l'hologramme codé dans la couche métallique 112 peut être reconstruite en couplant optiquement le dispositif émissif lumineux avec une lentille de Fourier selon le principe précédemment décrit en liaison avec la figure 9. En variante, une fonction de phase de l'hologramme synthétique codé dans la couche métallique 112 peut comporter une fonction optique sensiblement similaire à une fonction de convergence d'une lentille de Fourier, conformément au principe précédemment décrit en liaison avec la figure 10.

Il est également possible qu'une fonction de phase de l'hologramme synthétique codé dans la couche métallique 112 comporte une fonction optique de type lentille cylindrique apte à compenser une déformation du dispositif émissif lumineux 100, due à une déformation de la couche métallique 112 (et éventuellement de l'élément électriquement conducteur 104 lorsque le dispositif 100 comporte un tel élément) lors de l'échauffement de la couche métallique 112. L'hologramme synthétique codé permet ainsi de compenser un effet de courbure lié à la présence de contraintes sur les couches du dispositif émissif lumineux 100. Pour cela, en connaissant cet effet de courbure, l'hologramme est codé avec un effet de courbure inverse ce qui permet, lors de la reconstruction de l'image de l'hologramme, d'obtenir une annulation de l'effet de courbure. Cela se traduit par l'introduction d'une composante de phase de ce type de lentille cylindrique donnée par une équation similaire à l'équation (3) ci-dessus.

Par exemple, le dispositif lumineux 100 peut subir une déformation convexe selon l'axe x (axe représenté sur les figures 2 et 3) conduisant à une courbure de rayon égal à 1 mm. Dans ce cas, l'émission holographique est perturbée par un effet de lentille divergente de 1 mm de focale. Pour corriger cet effet, il est possible d'ajouter à la fonction de phase de l'hologramme une correction de phase convergente telle que $\Delta\phi(x) = \pi . x^2/(\lambda . f)$ avec f = 1 mm.

Compte tenu des motifs de diffraction réalisés dans la couche métallique 112, le couplage des ondes de surface s'effectue pour un couple donné de paramètres longueur d'onde / angle d'émission. La position de l'image holographique dans le plan de reconstruction dépend de manière similaire de la longueur d'onde comme indiqué dans l'équation (1).

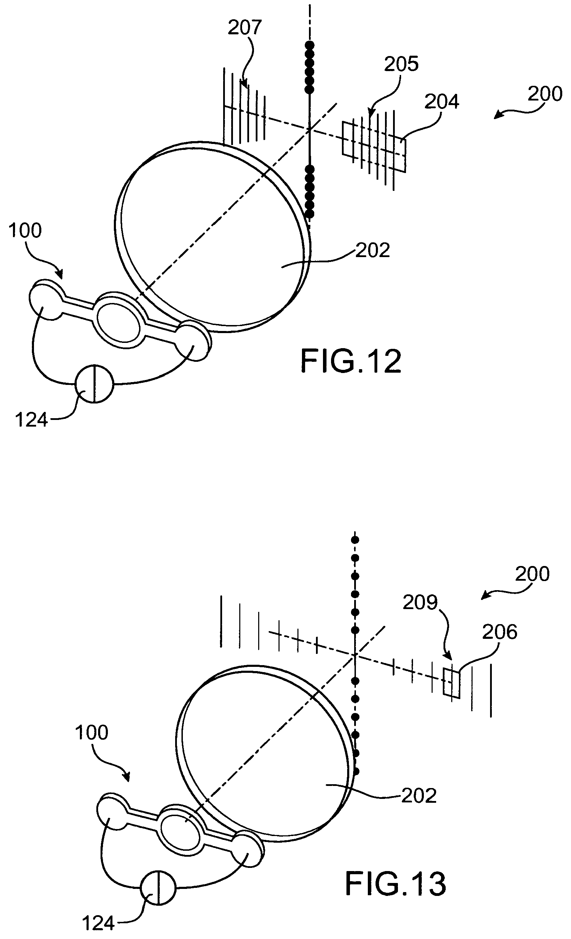

Un premier mode de réalisation d'un capteur de gaz 200 est décrit en liaison avec la figure 12. Le capteur de gaz 200 comporte le dispositif émissif lumineux 100 similaire à celui précédemment décrit en liaison avec la figure 2. Le dispositif émissif lumineux 100 comporte une lentille de Fourier 202 disposée à une distance focale objet de la couche métallique 112 du dispositif 100. Le capteur de gaz 200 comporte également un détecteur matriciel 204 (formé de plusieurs photodétecteurs disposés en matrice) disposé dans le plan de reconstruction de l'hologramme, à une distance focale image de la lentille de Fourier 202. Le dispositif 100 comporte en outre un générateur de courant 124 relié aux plots de connexion électrique de l'élément électriquement conducteur 104 afin de faire circuler un courant à travers l'élément électriquement conducteur 104 et provoquer ainsi un échauffement de la couche métallique 112. Lorsque le dispositif 100 ne comporte pas l'élément électriquement conducteur 104, le générateur de courant 124 peut être relié directement à la couche métallique 112 afin que celle-ci soit traversée par le courant généré par le générateur 124.

Dans ce premier exemple de réalisation, l'image de l'hologramme codé dans la couche métallique 112 du dispositif 100 correspond à une fente. Ainsi, la reconstruction de l'hologramme dans le plan de reconstruction donne l'image de plusieurs fentes 205 de différentes longueurs d'ondes disposées les unes à côté des autres et espacées latéralement les unes des autres. Le détecteur 204 est disposé dans la zone où se trouve l'ordre de diffraction 1 de l'hologramme. Des fentes 207 sont également présentes dans le zone où se trouvent l'ordre de diffraction -1, ou conjugué, de l'hologramme.

Le capteur de gaz 200 est destiné à réaliser une détection de présence de $CO_2$. La lumière émise par le dispositif émissif 100 traverse un espace (entre le dispositif 100 et le plan de reconstruction de l'hologramme où est disposé le détecteur 204) dans lequel se trouve le gaz à analyser. Compte tenu du pic d'absorption engendré par la présence de $CO_2$ dans le spectre lumineux (pic d'absorption à environ 4,25 $\mu$m), le détecteur 204 peut ainsi détecter aisément ce pic au niveau de l'image de la fente dont la bande spectrale inclut cette longueur d'onde du fait que cela se traduit par une fente d'intensité moindre que celle des autres fentes. Dans un tel capteur, la partie source émissive et la partie dispersion spectrale sont intégrées dans le dispositif émissif 100. De plus, la partie optique du capteur ne correspond qu'à une seule lentille de Fourier, ce qui réduit l'encombrement du capteur 200 par rapport aux capteurs de l'art antérieur faisant appel à plusieurs optiques de mise en forme du rayonnement lumineux.

Un deuxième mode de réalisation du capteur 200 est représenté sur la figure 13. Par rapport au capteur de gaz 200 précédemment décrit en liaison avec la figure 12, les paramètres de la lentille de Fourier 202 et de l'hologramme sont modifiés pour permettre une distribution latérale du spectre plus étendue, c'est-à-dire des fentes plus espacées les unes des autres. De plus, un seul photodétecteur 206 est positionné dans le plan de reconstruction de l'hologramme de manière à sélectionner la seule bande spectrale d'intérêt, c'est-à-dire la fente 209 correspondant à la zone spectrale d'absorption de l'espèce gazeuse à mesurer, ici le $CO_2$.

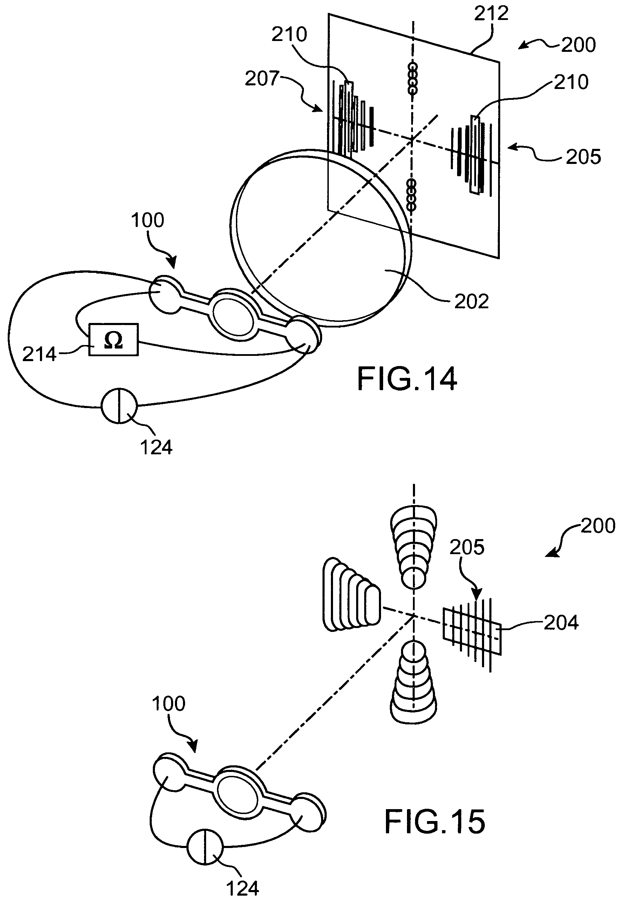

Un troisième mode de réalisation du capteur 200 est représenté sur la figure 14. Dans ce mode de réalisation, le détecteur comporte des bandes réfléchissantes 210 positionnées sur un fond absorbant 212, de part et d'autre de l'axe optique, c'est-à-dire dans les zones où se trouvent les ordres de diffraction 1 et -1 de l'hologramme.

Chacune des bandes 210 réfléchit une partie du spectre dispersé par l'hologramme. Le signal réfléchi est incident, en retour, sur le dispositif émissif 100, et est couplé par le mode de surface dans l'élément électriquement conducteur 104 ou, lorsque le dispositif 100 ne comporte pas l'élément électriquement conducteur 104, directement dans la couche métallique 112. Ce couplage provoque une légère augmentation de la température de l'élément électriquement conducteur 104 (ou de la couche métallique 112) qui peut être détectée par le changement de sa résistance électrique (en présence du pic d'absorption dû au $CO_2$, ce changement de résistance sera moins important). Un dispositif de mesure 214 de la résistance électrique de l'élément électriquement conducteur 104 (ou directement de la couche métallique 112) est donc relié électriquement aux deux plots de connexion électrique de l'élément électriquement conducteur 104 (ou de la couche métallique 112). La source incandescente du dispositif 100 joue donc également, dans ce mode de réalisation, une partie du rôle de détecteur du capteur 200.

Un quatrième mode de réalisation du capteur 200 est représenté sur la figure 15. Par rapport au capteur 200 précédemment décrit en liaison avec la figure 12, ce capteur 200 ne comporte pas la lentille de Fourier 202. L'hologramme est directement reconstruit dans le plan où se trouve le détecteur 204 du fait qu'une fonction de phase de l'hologramme synthétique codé dans la couche métallique 112 comporte une fonction optique correspondant à une fonction de convergence d'une lentille de Fourier. L'image holographique (fentes 205) apparait uniquement dans la zone correspondant à l'ordre de diffraction choisi, ici l'ordre 1 de l'hologramme. Un capteur de type matriciel 204 est positionné au niveau de l'image reconstruite pour détecter le spectre d'émission de la source lumineuse. En variante, le capteur de type matriciel 204 peut être remplacé par un photodétecteur 206 comme pour le capteur 200 décrit en liaison avec la figure 13. Dans un tel capteur, la partie source émissive, la partie de mise en forme optique et la partie de dispersion spectrale sont intégrées dans le dispositif émissif 100, ce qui réduit l'encombrement du capteur 200.

Selon un exemple de réalisation, la couche métallique 112 peut correspondre à une couche de tungstène d'épaisseur égale à environ 250 nm et en forme de disque de diamètre égal à environ 250 $\mu$m. Un hologramme de taille égale 80*80 pixels contenant un motif carré de 5*5 pixels est codé dans la couche métallique 112 avec un pas d'environ 3 $\mu$m et une profondeur d'environ 125 nm. Une focale de 25 mm est ajoutée à la fonction de phase radiale. Compte tenu du pic d'absorption à environ 4,25 $\mu$m, la fente est imagée à une distance longitudinale de 25 mm du dispositif 100 et à une distance latérale de 35 mm. Le motif carré holographique reproduit a une taille d'environ 2,2 mm dans le plan de reconstruction. On place donc à cet emplacement un détecteur de type PbSe de surface égale à environ 2x2 mm$^2$ de sensibilité maximale accordée à la longueur d'onde 4,2 $\mu$m (correspondant par exemple à un détecteur de type P9696-202 de la marque Hamamatsu®).

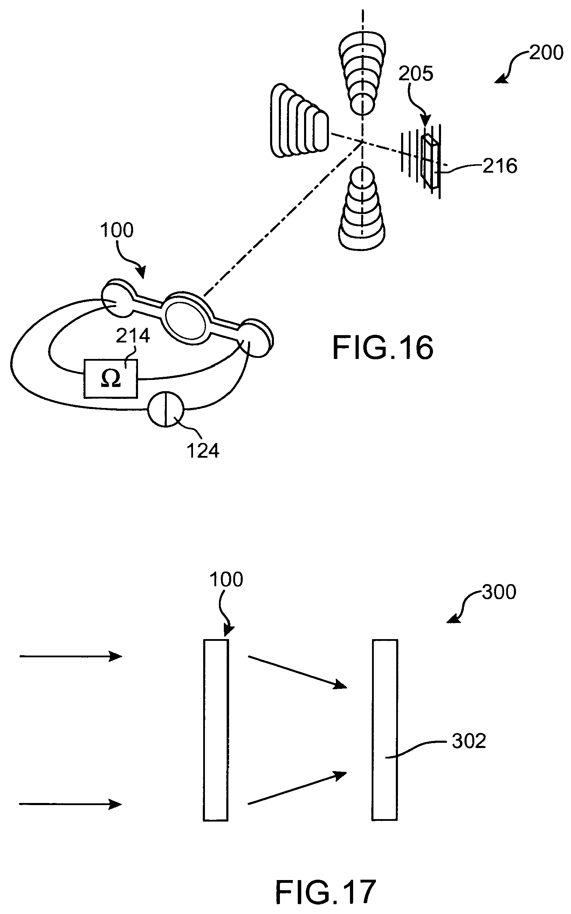

Un cinquième mode de réalisation du capteur 200 est représenté sur la figure 16. Comme dans le quatrième mode de réalisation décrit ci-dessus, le dispositif émissif 100 est réalisé tel qu'une fonction de phase de l'hologramme synthétique codé dans la couche métallique 112 comporte une fonction optique correspondant à une fonction de convergence d'une lentille de Fourier, permettant ainsi au capteur 200 d'être réalisé sans lentille de Fourier. De plus, dans ce cinquième mode de réalisation, seul un élément réfléchissant 216 est disposé dans le plan de reconstruction de l'hologramme, au niveau de l'ordre de diffraction choisi (ordre 1 dans cet exemple). Cet élément réfléchissant 216 n'est pas orienté parallèlement au plan de reconstruction, c'est-à-dire perpendiculairement à l'axe optique du capteur 200, comme dans le cas des bandes réflectrices 210 du capteur 200 précédemment décrit en liaison avec la figure 14.

L'élément réfléchissant 216 est orienté de manière à retourner la bande spectrale choisie dans la direction d'incidence, vers le dispositif émissif 100. Comme dans le troisième mode de réalisation précédemment décrit, un dispositif de mesure 214 de la résistance électrique de l'élément électriquement conducteur 104 (ou de la couche métallique 112) est relié électriquement aux deux plots de connexion électrique de l'élément électriquement conducteur 104 (ou de la couche métallique 112) afin de mesurer la présence du gaz à détecter via la mesure de la résistance électrique de l'élément électriquement conducteur 104 (ou de la couche métallique 112). Dans un tel capteur, la partie source émissive, la partie détection, la partie de mise en forme optique et la partie de dispersion spectrale sont intégrées dans le dispositif émissif 100, ce qui réduit considérablement l'encombrement du capteur 200.

Dans les exemples de réalisation précédemment décrits, l'image holographique est une fente mais d'autres distributions optimisant la détection spectrale destinée à être réalisée par le capteur peuvent être employées (par exemple une série de points).

Le dispositif émissif lumineux 100 précédemment décrit peut avantageusement être utilisé au sein d'un dispositif photovoltaïque 300 tel que représenté sur la figure 17. Selon les fonctions optiques codées dans l'hologramme, le dispositif peut comporter ou non une lentille de Fourier permettant de reconstruire l'hologramme transmis. Le dispositif photovoltaïque 300 comporte un élément de conversion photovoltaïque 302, par exemple une ou plusieurs cellules solaires, disposé dans le plan de reconstruction de l'hologramme, réalisant ainsi une conversion photovoltaïque de l'énergie reçue sous la forme de l'hologramme. Contrairement à l'application capteur de gaz précédemment décrite, l'échauffement de la couche métallique 112 du dispositif émissif 100 n'est pas obtenu en faisant circuler un courant électrique à travers cette couche ou à travers l'élément électriquement conducteur 104, mais par le biais d'un rayonnement solaire reçu par le dispositif 100. Du fait que la couche métallique 112 est sensible sur un large spectre, celle-ci s'échauffe puis réémet l'énergie suivant un rendement spectral adapté à celui de l'élément de conversion photovoltaïque 302, permettant ainsi d'améliorer l'absorption de rayonnement par l'élément de conversion photovoltaïque 302. Pour cette application, il est avantageux de coder une fonction optique de convergence dans l'hologramme, et/ou en codant l'image de l'élément de conversion photovoltaïque 302 en tant qu'hologramme, ce qui optimise le renvoi d'énergie sur l'élément de conversion photovoltaïque 302.

## Revendications

1. Dispositif émissif lumineux (100) comportant au moins :

   - une couche métallique (112) apte à s'échauffer et à propager des ondes de surface consécutives à l'échauffement de la couche métallique (112), la couche métallique (112) étant structurée telle qu'elle comporte plusieurs motifs de diffraction (40a-40d, 44a-44d, 120) aptes à réaliser une diffraction des ondes de surface vers des modes de propagation en espace libre, et dans laquelle un hologramme synthétique apte à contrôler la forme d'un rayonnement lumineux destiné à être émis par le dispositif émissif lumineux (100) via le contrôle d'un front d'onde destiné à être émis est codé tel que l'hologramme synthétique correspond à une image de phase codée en chaque pixel par un décalage de position d'un des motifs de diffraction (40a-40d, 44a-44d, 120) par rapport à une position de référence (122) dudit un des motifs de diffraction (40a-40d, 44a-44d, 120);
   - des moyens (104, 124) aptes à chauffer la couche métallique (112).

2. Dispositif émissif lumineux (100) selon la revendication 1, dans lequel, dans chaque pixel, l'image de phase est codée par un décalage, le long d'un premier axe, d'une position dudit un des motifs de diffraction (44a-44d, 120) par rapport à la position de référence (122) dudit un des motifs de diffraction (44a-44d, 120), les positions de référence (122) des motifs de diffraction (44a-44d, 120) étant définies comme correspondant à des intersections d'une grille de n lignes et de m colonnes régulièrement espacées les unes des autres, le premier axe étant parallèle aux n lignes ou aux m colonnes de la grille, les motifs de diffraction (44a-44d, 120) étant régulièrement espacés les uns des autres le long d'un deuxième axe perpendiculaire au premier axe, et chaque décalage de position d'un des motifs de diffraction (44a-44d, 120) par rapport à sa position de référence (122) est inférieur à une distance p séparant deux lignes ou deux colonnes adjacentes de la grille, avec n et m nombres entiers supérieurs à 1.

**3.** Dispositif émissif lumineux (100) selon la revendication 2, dans lequel les motifs de diffraction (44a-44d, 120) ont des dimensions sensiblement similaires les uns par rapport aux autres, et/ou dans lequel un rapport entre une dimension de chaque motif de diffraction (44a-44d, 120) selon le deuxième axe et une dimension dudit chaque motif de diffraction (44a-44d, 120) selon le premier axe est supérieur ou égal à environ 1,5, et/ou dans lequel les motifs de diffraction (40a-40d, 44a-44d, 120) comportent des ouvertures réalisées à travers au moins une partie de l'épaisseur de la couche métallique (112) et une surface d'une section d'une ou des ouvertures de chaque motif de diffraction (44a-44d, 120), dans un plan principal de la couche métallique (112), est supérieure ou égale à environ $p^2/2$.

**4.** Dispositif émissif lumineux (100) selon la revendication 1, dans lequel chaque motif de diffraction (40a-40d) forme une partie d'un réseau de diffraction comprenant plusieurs fentes parallèles les unes par rapport aux autres, et dans lequel, dans chaque pixel, l'image de phase est codée par un décalage, le long d'un premier axe, d'une position des parties des fentes dudit un des motifs de diffraction (40a-40d) par rapport à la position de référence dudit un des motifs de diffraction (40a-40d), les positions de référence des motifs de diffraction (40a-40d) étant définies comme correspondant aux positions dans lesquelles les parties des fentes de tous les motifs de diffraction (40a-40d) sont alignées les unes par rapport aux autres.

**5.** Dispositif émissif lumineux (100) selon la revendication 4, dans lequel les motifs de diffraction (40a-40d, 44a-44d, 120) comportent des ouvertures réalisées à travers au moins une partie de l'épaisseur de la couche métallique (112).

**6.** Dispositif émissif lumineux (100) selon l'une des revendications précédentes, dans lequel les moyens aptes à chauffer la couche métallique (112) comportent au moins un élément électriquement conducteur (104) couplé thermiquement avec la couche métallique (112) et apte à s'échauffer lorsqu'il est traversé par un courant électrique.

**7.** Dispositif émissif lumineux (100) selon l'une des revendications précédentes, comportant en outre un élément de support mécanique (102) sur lequel est disposée la couche métallique (112) et/ou, lorsque les moyens aptes à chauffer la couche métallique comportent l'élément électriquement conducteur (104), un élément isolant électrique (110) disposé entre l'élément électriquement conducteur (104) et la couche métallique (112).

**8.** Dispositif émissif lumineux (100) selon l'une des revendications précédentes, dans lequel les moyens aptes à chauffer la couche métallique (112) comportent au moins un dispositif (124) apte à faire circuler un courant électrique à travers la couche métallique (112) ou, lorsque lesdits moyens comportent l'élément électriquement conducteur (104), un dispositif (124) apte à faire circuler un courant électrique à travers l'élément électriquement conducteur (104).

**9.** Dispositif émissif lumineux (100) selon l'une des revendications précédentes, comportant en outre au moins une lentille de Fourier (202) disposée à une distance focale objet de la couche métallique (112) et à une distance focale image d'un plan destiné à recevoir l'émission lumineuse du dispositif (100).

**10.** Dispositif émissif lumineux (100) selon l'une des revendications 1 à 8, dans lequel une fonction de phase de l'hologramme synthétique codé dans la couche métallique (112) comporte une fonction optique sensiblement similaire à une fonction de convergence d'une lentille de Fourier.

**11.** Dispositif émissif lumineux (100) selon l'une des revendications précédentes, dans lequel une fonction de phase de l'hologramme synthétique codé dans la couche métallique (112) comporte une fonction optique apte à compenser une déformation du dispositif émissif lumineux (100) lors de l'échauffement de la couche métallique (112).

**12.** Dispositif émissif lumineux (100) selon l'une des revendications précédentes, dans lequel une image (205, 207, 209) de l'hologramme synthétique est une fente.

**13.** Capteur de gaz (200) comportant au moins un dispositif émissif lumineux (100) selon l'une des revendications précédentes et un détecteur (204, 206, 210, 214, 216) apte à détecter la présence d'au moins un gaz dans un espace traversé par au moins une image (205, 207, 209) de l'hologramme émise par le dispositif émissif lumineux (100).

**14.** Capteur de gaz (200) selon la revendication 13, dans lequel le détecteur (204, 206) est apte à réaliser une détection spectrale de l'image (205, 207, 209) de l'hologramme au moins dans une gamme de longueurs d'ondes comportant au moins une bande d'absorption spectrale du ou des gaz destiné(s) à être détecté(s) par le capteur (200).

**15.** Capteur de gaz (200) selon la revendication 14, dans lequel le détecteur (204) est apte à réaliser une détection spectrale de plusieurs images (205) de l'hologramme émis par le dispositif émissif lumineux (100) dans plusieurs gammes de longueurs d'ondes dont l'une au moins comporte la bande d'absorption spectrale du ou des gaz destiné(s) à être détecté(s) par le capteur (200), ou dans lequel le détecteur (206) est apte à réaliser une détection spectrale d'une seule image (209) de l'hologramme émis par le dispositif émissif lumineux (100) dans une seule gamme de longueurs d'ondes comportant la bande d'absorption spectrale du gaz destiné à être détecté par le capteur (200).

**16.** Capteur de gaz (200) selon la revendication 13, dans lequel le détecteur (210, 214, 216) comporte au moins :

   - un élément réfléchissant (210, 216) disposé en regard du dispositif émissif lumineux (100) et apte à refléter l'image (205) de l'hologramme ayant traversé l'espace comprenant le ou les gaz vers le dispositif émissif lumineux (100) ;
   - un dispositif de mesure (214) de la résistance électrique de la couche métallique (112) ou, lorsque les moyens aptes à chauffer la couche métallique (112) comportent l'élément électriquement conducteur (104), un dispositif de mesure (214) de la résistance électrique de l'élément électriquement conducteur (104).

**17.** Dispositif photovoltaïque (300) comportant au moins un dispositif émissif lumineux (100), le dispositif émissif lumineux (100) comportant au moins :

   - une couche métallique (112) apte à s'échauffer et à propager des ondes de surface consécutives à l'échauffement de la couche métallique (112), la couche métallique (112) étant structurée telle qu'elle comporte plusieurs motifs de diffraction (40a-40d, 44a-44d, 120) aptes à réaliser une diffraction des ondes de surface vers des modes de propagation en espace libre, et dans laquelle un hologramme synthétique apte à contrôler la forme d'un rayonnement lumineux destiné à être émis par le dispositif émissif lumineux (100) via le contrôle d'un front d'onde destiné à être émis est codé tel que l'hologramme synthétique correspond à une image de phase codée en chaque pixel par un décalage de position d'un des motifs de diffraction (40a-40d, 44a-44d, 120) par rapport à une position de référence (122) dudit un des motifs de diffraction (40a-40d, 44a-44d, 120);

   et dans lequel la couche métallique (112) du dispositif émissif lumineux (100) est apte à recevoir des rayons lumineux reçus par le dispositif photovoltaïque (300), et comportant en outre au moins un élément de conversion photovoltaïque (302) apte à recevoir une image de l'hologramme destinée à être émise par le dispositif émissif lumineux (100).

**Patentansprüche**

**1.** Lichtemissionsvorrichtung (100), umfassend wenigstens:

   - eine Metallschicht (112), die dazu ausgelegt ist, sich aufzuheizen und im Anschluss an die Aufheizung der Metallschicht (112) eine Ausbreitung von Oberflächenwellen zu ermöglichen, wobei die Metallschicht (112) derart strukturiert ist, dass sie mehrere Beugungsmuster (40a-40d, 44a-44d, 120) umfasst, die dazu ausgelegt sind, eine Beugung der Oberflächenwellen in Ausbreitungsmoden im freien Raum zu realisieren, und wobei ein synthetisches Hologramm, das dazu ausgelegt ist, die Form eines Lichtstrahls, der von der Lichtemissionsvorrichtung (100) emittiert werden soll, über die Steuerung einer Wellenfront zu steuern, die emittiert werden soll, derart codiert ist, dass das synthetische Hologramm einem Phasenbild entspricht, das in jedem Pixel durch einen Positionsversatz eines der Beugungsmuster (40a-40d, 44a-44d, 120) bezüglich einer Referenzposition (122) des einen der Beugungsmuster (40a-40d, 44a-44d, 120) codiert ist;
   - Mittel (104, 124), die dazu ausgelegt sind, die Metallschicht (112) aufzuheizen.

**2.** Lichtemissionsvorrichtung (100) nach Anspruch 1, bei der in jedem Pixel das Phasenbild durch einen Versatz, entlang einer ersten Achse, einer Position des einen der Beugungsmuster (44a-44d, 120) bezüglich der Referenzposition (122) des einen der Beugungsmuster (44a-44d, 120) codiert ist, wobei die Referenzpositionen (122) der Beugungsmuster (44a-44d, 120) definiert sind als den Schnitten eines Gitters von n Zeilen und von m Spalten entsprechend, die gleichmäßig voneinander beabstandet sind, wobei die erste Achse parallel zu den n Zeilen oder zu den m Spalten des Gitters ist, wobei die Beugungsmuster (44a-44d, 120) gleichmäßig voneinander entlang einer zweiten Achse orthogonal zur ersten Achse beabstandet sind, und wobei jeder Positionsversatz eines der Beugungsmuster (44a-44d, 120) bezüglich seiner Referenzposition (122) kleiner ist als ein Abstand p, der zwei benachbarte Zeilen oder Spalten des Gitters trennt, wobei n und m ganze Zahlen größer als 1 sind.

3. Lichtemissionsvorrichtung (100) nach Anspruch 2, bei der die Beugungsmuster (44a-44d, 120) Abmessungen haben, die zueinander im Wesentlichen ähnlich sind, und/oder bei der ein Verhältnis zwischen einer Abmessung jedes Beugungsmusters (44a-44d, 120) entlang der zweiten Achse und einer Abmessung des jeweiligen Beugungsmusters (44a-44d, 120) entlang der ersten Achse größer oder gleich ungefähr 1,5 ist, und/oder bei der die Beugungsmuster (40a-40d, 44a-44d, 120) Öffnungen umfassen, die durch wenigstens einen Teil der Dicke der Metallschicht (112) hindurch realisiert sind, und eine Oberfläche eines Schnitts einer oder der Öffnungen jedes Beugungsmusters (44a-44d, 120) in einer Hauptebene der Metallschicht (112) größer oder gleich ungefähr $p^2/2$ ist.

4. Lichtemissionsvorrichtung (100) nach Anspruch 1, bei der jedes Beugungsmuster (40a-40d) einen Teil eines Beugungsgitters bildet, das mehrere zueinander parallele Schlitze umfasst, und wobei in jedem Pixel das Phasenbild codiert ist durch einen Versatz, entlang einer ersten Achse, einer Position der Teile der Schlitze des einen Beugungsmusters (40a-40d) bezüglich der Referenzposition des einen der Beugungsmuster (40a-40d), wobei die Referenzpositionen der Beugungsmuster (40a-40d) definiert sind als den Positionen entsprechend, in denen die Teile der Schlitze aller Beugungsmuster (40a-40d) zueinander ausgerichtet sind.

5. Lichtemissionsvorrichtung (100) nach Anspruch 4, bei der die Beugungsmuster (40a-40d, 44a-44d, 120) Öffnungen umfassen, die durch wenigstens einen Teil der Dicke der Metallschicht (112) hindurch realisiert sind.

6. Lichtemissionsvorrichtung (100) nach einem der vorhergehenden Ansprüche, bei der die Mittel, die dazu ausgelegt sind, die Metallschicht (112) aufzuheizen, wenigstens ein elektrisch leitendes Element (104) umfassen, das thermisch mit der Metallschicht (112) gekoppelt und dazu ausgelegt ist, sich aufzuheizen, wenn es von einem elektrischen Strom durchflossen wird.

7. Lichtemissionsvorrichtung (100) nach einem der vorhergehenden Ansprüche, ferner umfassend ein mechanisches Stützelement (102), auf dem die Metallschicht (112) angeordnet ist, und/oder wenn die Mittel, die dazu ausgelegt sind, die Metallschicht aufzuheizen, das elektrisch leitende Element (104) umfassen, ein elektrisch isolierendes Element (110), das zwischen dem elektrisch leitenden Element (104) und der Metallschicht (112) angeordnet ist.

8. Lichtemissionsvorrichtung (100) nach einem der vorhergehenden Ansprüche, bei der die Mittel, die dazu ausgelegt sind, die Metallschicht (112) aufzuheizen, wenigstens eine Vorrichtung (124) umfassen, die dazu ausgelegt ist, einen elektrischen Strom durch die Metallschicht (112) fließen zu lassen, oder, wenn die Mittel das elektrisch leitende Element (104) umfassen, eine Vorrichtung (124), die dazu ausgelegt ist, einen elektrischen Strom durch das elektrisch leitende Element (104) hindurch fließen zu lassen.

9. Lichtemissionsvorrichtung (100) nach einem der vorhergehenden Ansprüche, ferner umfassend wenigstes eine Fourierlinse (202), die in einer Gegenstandsbrennweite der Metallschicht (112) und in einer Bildbrennweite einer Ebene angeordnet ist, die dazu ausgelegt ist, die Lichtemission der Vorrichtung (100) zu empfangen.

10. Lichtemissionsvorrichtung (100) nach einem der Ansprüche 1 bis 8, bei der eine Phasenfunktion des synthetischen Hologramms, das in der Metallschicht (112) codiert ist, eine optische Funktion umfasst, die im Wesentlichen ähnlich zu einer Konvergenzfunktion einer Fourierlinse ist.

11. Lichtemissionsvorrichtung (100) nach einem der vorhergehenden Ansprüche, bei der eine Phasenfunktion des synthetischen Hologramms, das in der Metallschicht (112) codiert ist, eine optische Funktion umfasst, die dazu ausgelegt ist, eine Verformung der Lichtemissionsvorrichtung (100) während des Aufheizens der Metallschicht (112) zu kompensieren.

12. Lichtemissionsvorrichtung (100) nach einem der vorhergehenden Ansprüche, bei der ein Bild (205, 207, 209) des synthetischen Hologramms ein Schlitz ist.

13. Gassensor (200), umfassend wenigstens eine Lichtemissionsvorrichtung (100) nach einem der vorhergehenden Ansprüche, sowie einen Detektor (204, 206, 210, 214, 216), der dazu ausgelegt ist, das Vorhandensein wenigstens eines Gases in einem Raum zu detektieren, der von wenigstens einem Bild (205, 207, 209) des Hologramms durchsetzt ist, das von der Lichtemissionsvorrichtung (100) emittiert wird.

14. Gassensor (200) nach Anspruch 13, bei dem der Detektor (204, 206) dazu ausgelegt ist, eine spektrale Detektion des Bilds (205, 207, 209) des Hologramms wenigstens in einem Wellenlängenbereich zu realisieren, der wenigstens ein spektrales Absorptionsband des Gases oder der Gase umfasst, das/die durch den Sensor (200) detektiert werden soll/sollen.

**15.** Gassensor (200) nach Anspruch 14, bei dem der Detektor (204) dazu ausgelegt ist, eine spektrale Detektion von mehreren Bildern (205) des Hologramms zu realisieren, das von der Lichtemissionsvorrichtung (100) in mehreren Wellenlängenbereichen emittiert wird, von denen wenigstens einer das spektrale Absorptionsband des Gases oder der Gase umfasst, die von dem Sensor (200) detektiert werden soll/sollen, oder bei dem der Detektor (206) dazu ausgelegt ist, eine spektrale Detektion eines einzigen Bilds (209) des Hologramms zu realisieren, das von der Lichtemissionsvorrichtung (100) in einem einzigen Wellenlängenbereich emittiert wird, der das spektrale Absorptionsband des Gases umfasst, das von dem Sensor (200) detektiert werden soll.

**16.** Gassensor (200) nach Anspruch 13, bei dem der Detektor (210, 214, 216) wenigstens umfasst:

- ein Reflektionselement (210, 216), das gegenüber der Lichtemissionsvorrichtung (100) angeordnet und dazu ausgelegt ist, das Bild (205) des Hologramms zur Lichtemissionsvorrichtung (100) zu reflektieren, das den Raum durchsetzt hat, der das Gas oder die Gase enthält;
- eine Vorrichtung (214) zum Messen des elektrischen Widerstands der Metallschicht (112), oder, wenn die Mittel, die dazu ausgelegt sind, die Metallschicht (112) aufzuheizen, das elektrisch leitende Element (104) umfassen, eine Vorrichtung (214) zum Messen des elektrischen Widerstands des elektrisch leitenden Elements (104).

**17.** Photovoltaikvorrichtung (300), umfassend wenigstens eine Lichtemissionsvorrichtung (100), wobei die Lichtemissionsvorrichtung (100) wenigstens umfasst:

- eine Metallschicht (112), die dazu ausgelegt ist, sich aufzuheizen und im Anschluss an die Aufheizung der Metallschicht (112) eine Ausbreitung von Oberflächenwellen zu ermöglichen, wobei die Metallschicht (112) derart strukturiert ist, dass sie mehrere Beugungsmuster (40a-40d, 44a-44d, 120) umfasst, die dazu ausgelegt sind, eine Beugung der Oberflächenwellen in Ausbreitungsmoden im freien Raum zu realisieren, und wobei ein synthetisches Hologramm, das dazu ausgelegt ist, die Form eines Lichtstrahls, der von der Lichtemissionsvorrichtung (100) emittiert werden soll, über die Steuerung einer Wellenfront zu steuern, die emittiert werden soll, derart codiert ist, dass das synthetische Hologramm einem Phasenbild entspricht, das in jedem Pixel durch einen Positionsversatz eines der Beugungsmuster (40a-40d, 44a-44d, 120) bezüglich einer Referenzposition (122) des einen der Beugungsmuster (40a-40d, 44a-44d, 120) codiert ist;

und wobei die Metallschicht (112) der Lichtemissionsvorrichtung (100) dazu ausgelegt ist, Lichtstrahlen zu empfangen, die durch die Photovoltaikvorrichtung (300) empfangen sind, und ferner umfassend wenigstes ein photovoltaisches Konversionselement (302), das dazu ausgelegt ist, ein Bild des Hologramms zu empfangen, das von der Lichtemissionsvorrichtung (100) emittiert werden soll.

## Claims

**1.** A light-emitting device (100) comprising at least:

- a metal layer (112) able to be heated and to propagate surface waves consecutive to the heating of the metal layer (112), the metal layer (112) being structured such that it comprises several diffraction patterns (40a-40d, 44a-44d, 120) able to carry out a diffraction of the surface waves to free-space propagation modes, and wherein a synthetic hologram able to control the form of a light radiation intended to be emitted by the light-emitting device (100) via controlling the wavefront intended to be emitted is coded such that the synthetic hologram corresponds to a phase image coded in each pixel by an offset of the position of one of the diffraction patterns (40a-40d, 44a-44d, 120) in relation to a reference position (122) of said one of the diffraction patterns (40a-40d, 44a-44d, 120);
- means (104, 124) able to heat the metal layer (112).

**2.** The light-emitting device (100) according to claim 1, wherein, in each pixel, the phase image is coded by an offset, along a first axis, of a position of said one of the diffraction patterns (44a-44d, 120) in relation to the reference position (122) of said one of the diffraction patterns (44a-44d, 120), wherein the reference positions (122) of the diffraction patterns (44a-44d, 120) are defined as corresponding to intersections of a grid of n lines and of m columns regularly spaced from each other, the first axis being parallel to the n lines or to the m columns of the grid, wherein the diffraction patterns (44a-44d, 120) are regularly spaced from each other along a second axis perpendicular to the first axis, and wherein each offset in position of one of the diffraction patterns (44a-44d, 120) in relation to its reference position (122) is less than a distance p that separates the two adjacent lines or two adjacent columns of the grid, with n and m integers greater than 1.

3. The light-emitting device (100) according to claim 2, wherein the diffraction patterns (44a-44d, 120) have dimensions that are substantially similar in relation to each other, or wherein a relation between a dimension of each diffraction pattern (44a-44d, 120) according to the second axis and a dimension of said each diffraction pattern (44a-44d, 120) according to the first axis is greater than or equal to about 1.5, and/or wherein the diffraction patterns (40a-40d, 44a-44d, 120) comprise openings made through at least a part of the thickness of the metal layer (112) and a surface of a section of one opening or of the openings of each diffraction pattern (44a-44d, 120), in a main plane of the metal layer (112), is greater than or equal to about $p^2/2$.

4. The light-emitting device (100) according to claim 1, wherein each diffraction pattern (40a-40d) forms a part of a diffraction grating that comprises several slots parallel to one another, wherein, in each pixel, the phase image is encoded by an offset, along a first axis, of a position of the parts of the slots of said one of the diffraction patterns (40a-40d) in relation to the reference position of said one of the diffraction patterns (40a-40d), and wherein the reference positions of the diffraction patterns (40a-40d) are defined as corresponding to the positions wherein the parts of the slots of all of the diffraction patterns (40a-40d) are aligned in relation to each other.

5. The light-emitting device (100) according to claim 4, wherein the diffraction patterns (40a-40d, 44a-44d, 120) comprise openings made through at least a part of the thickness of the metal layer (112).

6. The light-emitting device (100) according to one of previous claims, wherein the means able to heat the metal layer (112) comprise at least one electrically conductive element (104) thermally coupled with the metal layer (112) and able to be heated when an electric current passes through it.

7. The light-emitting device (100) according to one of previous claims, further comprising a mechanical support element (102) whereon is arranged the metal layer (112) and/or, when the means able to heat the metal layer comprise the electrically conductive element (104), an electric insulating element (110) arranged between the electrically conductive element (104) and the metal layer (112).

8. The light-emitting device (100) according to one of previous claims, wherein the means able to heat the metal layer (112) comprise at least one device (124) able to circulate an electric current through the metal layer (112) or, when said means comprise the electrically conductive element (104), a device (124) able to circulate an electric current through the electrically conductive element (104).

9. The light-emitting device (100) according to one of previous claims, further comprising at least one Fourier lens (202) arranged at a front focal distance from the metal layer (112) and at a rear focal distance from a plane intended to receive the light emission from the device (100).

10. The light-emitting device (100) according to one of claims 1 to 8, wherein a phase function of the synthetic hologram encoded in the metal layer (112) comprises an optic function that is substantially similar to a convergence function of a Fourier lens.

11. The light-emitting device (100) according to one of previous claims, wherein a phase function of the synthetic hologram encoded in the metal layer (112) comprises an optic function able to compensate for a deformation of the light-emitting device (100) during the heating of the metal layer (112).

12. The light-emitting device (100) according to one of previous claims, wherein an image (205, 207, 209) of the synthetic hologram is a slot.

13. A gas sensor (200) comprising at least one light-emitting device (100) according to one of previous claims and a detector (204, 206, 210, 214, 216) able to detect the presence of at least one gas in a space through which passes at least one image (205, 207, 209) of the hologram emitted by the light-emitting device (100).

14. The gas sensor (200) according to claim 13, wherein the detector (204, 206) is able to carry out a spectral detection of the image (205, 207, 209) of the hologram at least in one range of wavelengths comprising at least one spectral absorption band of the gas or gases intended to be detected by the sensor (200).

15. The gas sensor (200) according to claim 14, wherein the detector (204) is able to carry out a spectral detection of several images (205) of the hologram emitted by the light-emitting device (100) in several ranges of wavelengths of which at least one comprises the spectral absorption band of the gas or gases intended to be detected by the sensor (200), or wherein the detector (206) is able to carry out a spectral detection of a single image (209) of the hologram emitted by the light-emitting device (100) in a single range of wavelengths comprising the spectral absorption band of the gas intended to be detected by the sensor (200).

16. The gas sensor (200) according to claim 13, wherein the detector (210, 214, 216) comprises at least:

- a reflective element (210, 216) arranged facing the light-emitting device (100) and able to reflect the image (205) of the hologram that has passed through the space comprising the gas or gases towards the light-emitting device (100);
- a device (214) for measuring the electrical resistance of the metal layer (112) or, when the means able to heat the metal layer (112) comprises the electrically conductive element (104), a device (214) for measuring the electrical resistance of the electrically conductive element (104).

17. A photovoltaic device (300) comprising at least one light-emitting device (100), wherein the light-emitting device (100) comprises at least:

- a metal layer (112) able to be heated and to propagate surface waves consecutive to the heating of the metal layer (112), the metal layer (112) being structured such that it comprises several diffraction patterns (40a-40d, 44a-44d, 120) able to carry out a diffraction of the surface waves to free-space propagation modes, and wherein a synthetic hologram able to control the form of a light radiation intended to be emitted by the light-emitting device (100) via controlling the wavefront intended to be emitted is coded such that the synthetic hologram corresponds to a phase image coded in each pixel by an offset of the position of one of the diffraction patterns (40a-40d, 44a-44d, 120) in relation to a reference position (122) of said one of the diffraction patterns (40a-40d, 44a-44d, 120);

and wherein the metal layer (112) of the light-emitting device (100) is able to receive light rays received by the photovoltaic device (300), and further comprising at least one photovoltaic conversion element (302) able to receive an image of the hologram intended to be emitted by the light-emitting device (100).

FIG.1

FIG.2

EP 2 846 201 B1

FIG.3

FIG.4

FIG.5

FIG.6

FIG.7

x

y

Ordre 1
hologramme

Ordre -1
réseau

Ordre 0

Ordre 1
réseau

Ordre -1
hologramme

## FIG.8

112

38

36

$\alpha$

$\Delta$

$f$

$f$

## FIG.9

112

36

$\alpha$

$\Delta$

$f$

## FIG.10

FIG.11

207

205

204

200

100

202

124

**FIG.12**

200

209

206

100

202

124

**FIG.13**

FIG.14

FIG.15

FIG.16

FIG.17

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **P. BARRITAULT et al.** Mid-IR source based on a free-standing microhotplate for autonomous CO2 sensing in indoor applications. *ensors and Actuators A: Physical,* Décembre 2011, vol. 172, 379-385
- **F. MARQUIER et al.** Coherent spontaneous emission of light by thermal sources. *Physical Review B,* 2004, vol. 69, 155412
- **M. LAROCHE et al.** Highly directional radiation generated by a tungsten thermal source. *Optics Letters,* 2005, vol. 30, 2623-2625
- **I. PUSCASU et al.** Photonic crystals enable infrared gas sensors. *Nanoengineering: Fabrication, Properties, Optics, and Devices. Edited by Dobisz, Elizabeth A.; Eldada, Louay A. Proceedings of the SPIE,* 2004, vol. 5515, 58-66
- **WAI-HON LEE.** Binary computer-generated holograms. *Applied Optics,* 01 Novembre 1979, vol. 18 (21
- **A.W. LOHMANN ; D.P. PARIS.** Binary Fraunhofer Holograms, Generated by Computer. *Appl. Opt.,* 1967, vol. 6, 1739-1748
- **C.B. BURCKHARDT.** Use of a random phase mask for the recording of Fourier transform holograms of data masks. *Appl. Opt.,* 1970, vol. 9 (3), 695-700